# DEMANDE DE BREVET EUROPEEN

(11) **EP 0 534 814 A1**
(43) Date de publication de la demande: **31.03.1993**
(21) Numéro de dépôt: 92402442.5
(22) Date de dépôt: 07.09.1992
(51) Int. Cl.: C07D 471/04, A61K 31/435

(54) **Procédé de préparation de l'isomère dextrogyre d'un dérivé de l'isoindolinone**

(30) Priorité: 09.09.1991 FR 9111101
(71) Demandeur: RHONE-POULENC RORER S.A., 92160 Antony (FR)
(72) Inventeur: Busson, André, F-91230 Montgeron (FR); Daubie, Christophe, F-75005 Paris (FR)
(74) Mandataire: Pilard, Jacques

(57) **Abrégé**

Procédé de préparation de l'isomère dextrogyre du produit de formule (I) par désalcoxycarbonylation d'un ester de menthyle optiquement actif de formule (II).

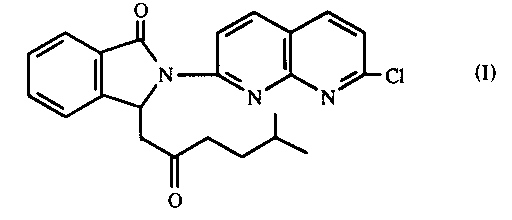

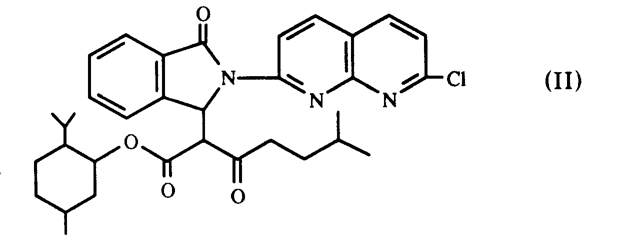

## Description

La présente invention concerne un procédé de préparation de l'isomère dextrogyre du dérivé de l'isoindolinone de formule: 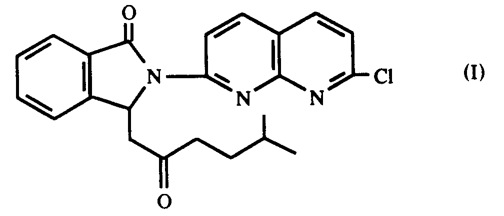
qui présente des propriétés anxiolytiques, hypnotiques, anticonvulsivantes, antiépileptiques et myorelaxantes remarquables.

Selon le brevet américain US 4 960 779, le produit de formule (I) dextrogyre peut être obtenu à partir du racémique correspondant par chromatographie sur phase chirale. Cependant l'application industrielle de ce procédé n'est pas toujours de réalisation commode.

Il a maintenant été trouvé, et c'est ce qui fait l'objet de la présente invention, que l'isomère dextrogyre du produit de formule (I) peut être obtenu par désalcoxycarbonylation d'un ester optiquement actif de formule :

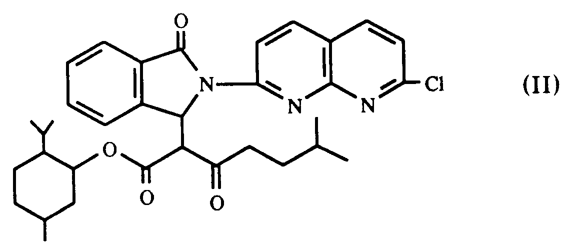

Généralement, la désalcoxycarbonylation de l'ester de menthyle de formule (II) est effectuée par action d'un acide minéral fort à une température voisine de 0°C. Il est particulièrement avantageux, peut obtenir une énantiosélectivité satisfaisante, d'utiliser l'acide sulfurique concentré.

L'ester de menthyle de formule (II) peut être obtenu par action du β-cétoester de formule : 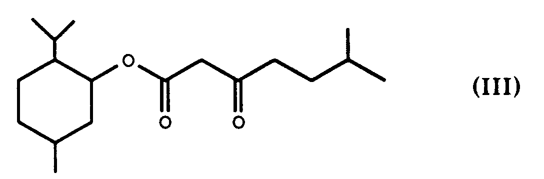
sur le dérivé de lisoindolinone de formule :

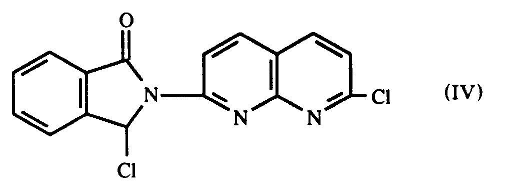

La condensation s'effectue en général dans un solvant organique en présence d'une base. Comme solvant organique, on utilise généralement le diméthylformamide, l'acétonitrile, le diméthylsulfoxyde, les hydrocarbures aromatiques (benzène, toluène) ou la N-méthylpyrrolidone. Comme base, on utilise généralement un carbonate de métal alcalin (sodium, potassium) ou un hydrure tel que l'hydrure de sodium. Généralement, la réaction est mise en oeuvre à une température comprise entre 0 et 50°C.

Le produit de formule (IV) peut être obtenu par chloruration du produit de formule:

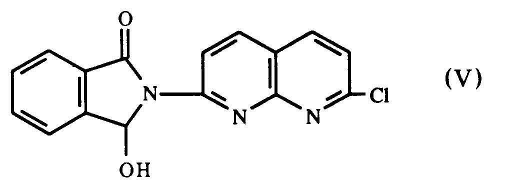

On opère généralement en présence d'un agent de chloruration tel que le chlorure de sulfinylé ou l'oxychlorure de phosphore en présence de quantités catalytiques de diméthylformamide à une température comprise entre 20°C et la température de reflux du mélange réactionnel.

Le produit de formule (V) peut être obtenu selon le procédé décrit, par exemple, dans le brevet belge BE 835 325.

Le β-cétoester de formule (III) peut être obtenu selon le procédé décrit par D.F. Taber et J.C. Amedio Jr., J. Org. Chem., 50, 3618 (1985) pour la préparation de β-cétoester par transestérification catalysée à la diméthylaminopyridine.

L'exemple suivant illustre la présente invention.

### EXEMPLE

A une solution de 3,3 g de chloro-3 (chloro-7 naphtyridine-1,8 yl-2)-2 isoindolinone-1 dans 33 cm3 de N-méthylpyrrolidinone, on ajoute, sous agitation, 3,72 g de méthyl-6 oxo-3 heptanoate de menthyle et 3,45 g de carbonate de potassium. La suspension est maintenue pendant 12 heures à une température voisine de 20°C. A la solution obtenue on ajoute 75 cm3 d'eau et on extrait par deux fois 25 cm3 de dichlorométhane. Les extraits organiques réunis sont lavés par 3 fois 50 cm3 d'eau, séchés sur sulfate de magnésium, filtrés et concentrés à sec sous pression réduite (20 kPa) à une température voisine de 40°C. On obtient ainsi 6 g de [(chloro-7 naphtyridine-1,8 yl-2)-2 oxo-3 isoindolinyl-1]-2 méthyl-6 oxo-3 heptanoate de menthyle dont on sépare l'isomère dextrogyre par chromatographie liquide préparative à haute performance sur colonne Kromasil Si 100 Å de 4 x 25 cm en utilisant comme phase mobile le mélange hexane-méthyl t.butyléther (90-10 en volumes) au débit de 100 cm3/minute.

A 5 cm3 d'acide sulfurique à 98 % on ajoute, sous agitation à 0°C, 50 mg de (+)- [(chloro-7 naphtyridine-1,8 yl-2)-2 oxo-3 isoindolinyl-1]-2 méthyl-6 oxo-3 heptanoate de menthyle. {[α]²⁰_{D} = + 233,9° (c = 1 ; chlorure de méthylène)}. La solution obtenue est agitée pendant 1 heure à une température voisine de 0°C, puis dans 25 g de glace. On extrait la phase aqueuse par deux fois 5 cm3 de dichlorométhane. Les extraits organiques réunis sont lavés par 10 cm3 d'une solution diluée d'ammoniaque (q.s.p. pH = 7), séchés sur sulfate de magnésium, filtrés et concentrés à sec sous pression réduite (20 kPa) à une température voisine de 20°C. On obtient ainsi 32,3 mg de (+)-(chloro-7 naphtyridine-1,8 yl-2)-2 (méthyl-5 oxo-2 hexyl)-3 isoindolinone-1 sous forme d'un solide blanc dont les caractéristiques sont les suivantes :
- titre énantiomérique : 95 %
- pouvoir rotatoire: [α]²⁰_{D} = +123° (c = 1 ; CH₂Cl₂).

## Revendications

**1 -** Procédé de préparation de l'isomère dextrogyre du produit de formule: 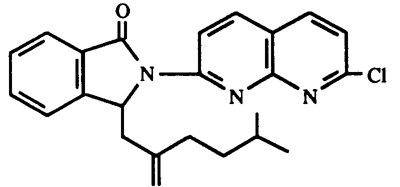 caractérisé en ce que l'on effectue une désalcoxycarbonylation de l'ester de menthyle dextrogyre de formule:

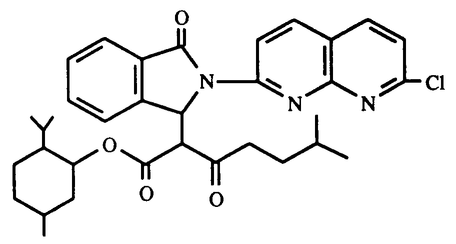

**2 -** Procédé selon la revendication 1 caractérisé en ce que la désalcoxycarbonylation est effectuée au moyen d'un acide fort.

**3 -** Procédé selon la revendication 2 caractérisé en ce que l'acide fort est l'acide sulfurique.

**4 -** Procédé selon l'une des revendications 1 à 3 caractérisé en ce que l'on opère à une température voisine de 0°C.
